# EUROPEAN PATENT SPECIFICATION

(11) **EP 4 021 351 B1**
(45) Date of publication and mention of the grant of the patent: **20.03.2024**
(21) Application number: 20790062.2
(22) Date of filing: 01.10.2020
(51) Int. Cl.: A61F 2/24

(54) **DOCKING ELEMENT**
ANDOCKELEMENT
ÉLÉMENT D'ANCRAGE

(30) Priority: 03.10.2019 US 201962909787 P
(43) Date of publication of application: 06.07.2022
(73) Proprietor: TruLeaf Medical Ltd., Or Akiva 3063908 (IL)
(72) Inventor: BENICHOU, Netanel, 3080800 D.N. Hof Carmel (IL); SPENSER, Benjamin, 3099200 D.N. Hof Carmel (IL); SOBOL, Moran, 3433410 Haifa (IL)
(74) Representative: Wright, Howard Hugh Burnby
(86) International application number: PCT/IB2020/059195
(87) International publication number: WO 2021/064624

(56) References cited:
- WO-A1-2018/178966
- US-A1- 2014 243 966
- US-A1- 2019 209 320

## Description

### FIELD OF EMBODIMENTS OF THE INVENTION

Some applications of the present invention generally relate to medical apparatus and methods. Specifically, some applications of the present invention relate to apparatus for use with a prosthetic mitral valve.

### BACKGROUND

Atrioventricular valves are cardiac valves that prevent backflow from the ventricles into the atria during systole. They are anchored to the wall of the heart at the fibrous skeleton by anchoring tendons named chordae tendineae. The chordae tendineae are attached to papillary muscles. Together, the papillary muscles and the chordae tendineae keep the valves from prolapsing into the atria when they close during systole. The actual opening and closing of the valves is caused by a pressure gradient across the valve. The left-side atrioventricular valve is a bicuspid valve having two leaflets, and is commonly known as the mitral valve. The right-side atrioventricular valve is a tricuspid valve, having three leaflets. Both of these valves may be damaged and dysfunctional, resulting in leakage during systole, requiring the valves to be repaired or replaced.

While the mitral valve is generally an ellipse or D-shaped, the tricuspid valve is more circular. The left ventricle pumps oxygenated blood around the body and so the mitral valve has to withstand a higher pressure than the tricuspid valve which only has to pump deoxygenated blood to the nearby lungs.

Occasionally, the mitral valve is congenitally abnormal or destroyed by infection or a bacterial endocarditis. More often, the mitral valve becomes degenerative with age, or as a result of rheumatic fever. There are different valvular heart disorders associated with the mitral valve such as mitral stenosis and mitral regurgitation. In the case of mitral stenosis, the valve orifice, i.e., the cross-section available for blood passage is reduced because of calcium nodes, leaflet thickening and/or reduced leaflet mobility, and, consequently, the valve does not allow normal blood flow. To overcome the damaged valve and to transport the same amount of blood, the left atrium requires a higher pressure than normal. The constant pressure overload of the left atrium may cause it to increase in size and become more prone to develop atrial fibrillation and to lose the atrial kick. The loss of the atrial kick due to atrial fibrillation can cause a precipitous decrease in cardiac output. A reduction in cardiac output, associated with acceleration of heart rate and shortening of the diastolic time, frequently leads to congestive heart failure. In most cases, mitral stenosis is due to rheumatic heart disease. The treatment options for mitral stenosis include medical management, surgical repair, surgical replacement of the valve, and percutaneous balloon valvuloplasty.

Mitral regurgitation causes heart murmurs and may have severe physiological consequences. Mitral regurgitation is caused either by ischemic heart disease (such cases being called "ischemic mitral regurgitation"), or mitral valve prolapse. Ischemic mitral regurgitation is a result of ventricular remodeling which is secondary to ischemic heart disease. The heart's posterior wall, which is not attached to the heart's fibrous skeleton, dilates. As a result of the change of the left ventricular geometry, the posterior leaflet, which is attached to the posterior heart wall, is displaced and misaligned from the anterior leaflet which results in mitral regurgitation.

Mitral valve prolapse is a condition caused by degeneration of the valve's connective tissue. Patients with classic mitral valve prolapse have surplus connective tissue. This weakens the leaflets and adjacent tissue, resulting in increased leaflet area and elongation of the chordae tendineae. Elongation of the chordae tendineae often causes rupture. Tweaked leaflets may be displaced in some portion of one or both of the abnormally thickened mitral valve leaflets into the left atrium during systole. Advanced lesions lead to leaflet folding, inversion, and displacement toward the left atrium. The abnormal leaflet structure leads to incomplete closure of the mitral valve and consequent mitral regurgitation.

In mitral regurgitation, the heart has to work harder by pumping not only the regular volume of blood, but also the extra volume of blood that is regurgitated back into the left atrium. The added workload creates an excessive strain on the left ventricle, which can lead to heart failure.

While patients with mild to moderate mitral regurgitation caused by mitral valve prolapse might experience no symptoms, increasing severity, even without symptoms, increases the load on the left ventricle. Over time this can result in ventricular dilatation and congestive heart failure.

Mitral valve disease is conventionally treated by open heart surgery; either by surgical repair, which is usually performed using an annuloplasty ring, or by surgical replacement with a valve prosthesis. In some cases, such as when the valve is too damaged, mitral valves may require replacement. Mitral valve replacement may be performed robotically or manually. Surgical valve replacement or repair is often a demanding operation as it requires cardiopulmonary bypass and it can expose patients, especially elderly ones, to many risks.

A large variety of percutaneous or transcutaneous medical procedures are currently being developed and/or practiced. For example, transcatheter procedures are known for replacement of aortic and pulmonary heart valves. These procedures, which are performed under local anesthesia in the cardiac catheterization lab, rather than by cardiac surgery, offer benefits to these patients. According to such approaches, the valve is inserted on a delivery device similar to a catheter or a sheath and then implanted in the desired location via access through a large blood vessel such as the femoral artery, for example. This involves making a very small perforation in the patient's skin, such as in the groin area, in order to access the femoral artery. This minimally invasive option is usually safer than open heart surgery, and recovery times are typically shorter.

WO2018178966 A1 discloses an apparatus for treating a subject with a diseased mitral valve. A docking element is implanted within the subject's left atrium such that no portion of the docking element extends through the subject's mitral valve. The docking becomes anchored to tissue of the left atrium at least partially via ingrowth of the tissue of the left atrium. The docking element (includes a ring, which is implanted at the subject's mitral valve annulus, and a frame, having a height of at least 15 mm, which extends upwardly from the ring. A prosthetic mitral valve apparatus is placed at least partially inside the docking element subsequent to the ingrowth of the tissue of the left atrium having occurred, and becomes anchored to the docking element, at least partially by radially expanding against the ring. Other applications are also described.

### SUMMARY OF EMBODIMENTS

In accordance with some applications of the present invention, a docking element is configured to facilitate anchoring of a prosthetic mitral valve apparatus to a subject's mitral valve. The docking element is typically disposed, in its entirety, within the left atrium, and does not protrude into the left ventricle.

Typically, the prosthetic mitral valve apparatus is a stented valve that comprises a stented frame that is configured to support prosthetic valve leaflets. Typically, the stented frame is covered with a covering material (e.g., a fabric such as PET, PTFE, and/or nylon).

The prosthetic mitral valve apparatus is typically implanted within the native mitral valve of a subject with a diseased native valve, and the prosthetic valve leaflets function such as to replace the functioning of the native valve leaflets. Typically, the prosthetic valve leaflets are configured to act as a one-way valve, whereby in their open positions with respect to one another the leaflets allow flow to pass through the prosthetic valve apparatus from the inlet (on the atrial side) to the outlet (on the ventricular side), whereas reverse flow is prevented due to collapsible slack portions of the valve leaflets collapsing inwardly to block the reverse flow.

Typically, the docking element is implanted within the left atrium more than one week, or more than one month, before the implantation of the prosthetic mitral valve apparatus. Subsequent to the implantation of the docking element, and before the implantation of the prosthetic mitral valve apparatus, the anchoring of the docking element is typically strengthened by virtue of tissue ingrowth that occurs around the docking element, e.g., as described hereinbelow. Typically, no portion of the docking element extends through the subject's native mitral valve. Further typically, by virtue of the fact that no portion of the docking element extends through the subject's native mitral valve, the native mitral valve leaflets are able to continue functioning in their normal manner subsequent to the implantation of the docking element, and prior to the implantation of the prosthetic mitral valve apparatus.

For some applications, the docking element includes a frame and a ring coupled to the frame. Typically, in its deployed state inside the left atrium, the ring is disposed transversely with respect to the frame, such that a plane defined by the ring is substantially parallel to the longitudinal axis of the frame. For some applications, the ring is configured to be disposed at a height that is level with, slightly above, or slightly below the mitral annulus, and the outer perimeter of the ring is smaller than the perimeter defined by the mitral annulus, such that the ring "floats" above the native valve leaflets. Such applications of the present invention are described in further detail hereinbelow.

Typically, the frame is made of a self-expandable, shape memory material (e.g., a shape memory alloy, such as nitinol), that is cut and shape set such as to define a plurality of cells defined by struts of the shape memory material. Alternatively or additionally, the frame is assembled from wires that are made of a self-expandable, shape memory material (e.g., a shape memory alloy, such as nitinol). Typically, a portion of the frame that is closest to the ring is covered in a skirt of a material that facilitates tissue ingrowth to the frame. For example, the skirt may be made of a fabric such as PET, PTFE, and/or nylon, and may be coupled to the outside of the frame and/or the ring using stitches. Typically, the portion of the frame that is configured to be disposed in the vicinity of the mitral annulus ( e.g., at the mitral annulus, and/or slightly above (e.g., within 15 mm of) the mitral annulus)) is covered with the skirt. For some applications, the skirt is configured to be disposed from between 1 mm and 2 mm above the mitral annulus (or from the height of the annulus) until a height of between 15 mm and 20 mm above the mitral annulus.

The docking element includes a radial protrusion that extends radially from the frame, typically above the ring and/or level with the ring. For example, the radial protrusion may be shaped as a flap and/or a torus that is disposed around the circumference of the frame. The protrusion is configured to contact the walls of the left atrium, and to promote tissue ingrowth from the walls of the left atrium to the protrusion. For some applications, the radial protrusion is configured to conform to the shape of the atrial walls in the vicinity of the mitral annulus, such that the protrusion fills any gaps between the outside of the frame and atrial walls in the vicinity of the mitral annulus. Thus, the docking element typically becomes anchored to the walls of the left atrium by virtue of the tissue ingrowth. For some applications, the protrusion is configured to act as a bridge for the tissue ingrowth, with the protrusion being configured to encourage tissue ingrowth from the walls of the left atrium, across the protrusion, and into the skirt and/or into the frame. Typically, the protrusion is configured to contact the walls of the left atrium in the vicinity of the mitral annulus, e.g., at the height of the mitral annulus, and/or from between 1 mm and 2 mm above the mitral annulus until a height of between 15 mm and 20 mm above the mitral annulus.

Typically, the outer perimeter of the ring is smaller than the size of the inner perimeter of the radial protrusion. There is a bridging material (e.g., a fabric) that bridges and forms a seal between the ring and the radial protrusion. Typically, the bridging material that bridges between the ring and the radial protrusion is a portion of the skirt. In accordance with the above description, the docking element includes a combination of three components: 1) the radial protrusion, which is configured to become anchored to the tissue in the vicinity of the mitral annulus, via tissue ingrowth, 2) the ring, to which the prosthetic mitral valve apparatus is configured to become anchored, and 3) a bridging material that bridges and forms a seal between these two elements.

For some applications, the radial protrusion is self-expandable and includes a coiled spring (which is typically made of a self-expandable metal or alloy, such a nitinol) covered with a fabric (such as PET, PTFE, and/or nylon) that is configured to promote tissue ingrowth. In accordance with respective embodiments, the coiled spring is a round coil, a triangular coil, or a differently shaped coil. The coiled spring is configured to cause the self-expandable protrusion to extend radially outwardly from the outer surface of the frame, such that the protrusion conforms with the anatomy of the left atrium in regions in which the frame itself is not compliant enough to do so. In this manner, the self-expandable protrusion fills the gaps between the outside of the frame and the walls of the left atrium.

Typically, the radial protrusion extends radially outwardly from the outer surface of the frame around more than 70 percent (e.g., more than 90 percent) of the circumference of the frame. Further typically, the protrusion extends radially outwardly from the outer surface of the frame around the full circumference of the frame.

For some applications, the docking element includes the frame, the ring, the skirt and the radial protrusion. The radial protrusion is configured to extend to the walls of the left atrium in the vicinity of (e.g., at the height of or slightly above (e.g., within 15 mm of)) the native mitral annulus, and to encourage tissue ingrowth from the subject's left atrium to the docking element, e.g., tissue ingrowth to the radial protrusion itself, and/or tissue ingrowth via the radial protrusion to the frame and/or the skirt. A portion of the skirt acts as a bridging material and bridges and seals between the radial protrusion and the ring, such that the bridging material occupies and seals some of the area defined by the native mitral annulus. Thus, the ring acts as an artificial mitral annulus that is smaller than the native mitral annulus.

There is therefore provided, in accordance with some applications of the present invention, apparatus for treating a subject with a diseased mitral valve, the apparatus including:
a docking element configured to be implanted within a left atrium of the subject such that no portion of the docking element extends through the subject's mitral valve, the docking element comprising:
   a ring;
   a frame extending upwardly from the ring, the frame being configured to anchor the docking element within the left atrium, prior to tissue ingrowth to the docking element occurring, by the frame expanding against at least one of walls of the left atrium and a roof of the left atrium;
   a radial protrusion configured to extend radially outwardly from the frame, to be disposed in a vicinity of the subject's native mitral annulus, and to generate tissue ingrowth to the radial protrusion from walls of the left atrium at least in the vicinity of the subject's native mitral annulus; and
   a bridging material disposed between radial protrusion and the ring, the bridging material forming a seal between the radial protrusion and the ring; and
a prosthetic mitral valve apparatus configured:
   subsequent to the ingrowth of the tissue from walls of the left atrium to the radial protrusion having occurred, to be placed at least partially inside the docking element, and
   to become anchored to the docking element, at least partially by radially expanding against the ring.

In some applications, the radial protrusion is configured to extend radially outwardly from the frame at a height that is level with the ring.

In some applications, the radial protrusion is configured to extend radially outwardly from the frame at a height that is between 1 mm and 20 mm above the ring.

In some applications, the radial protrusion is configured to conform to the shape of the walls of the left atrium in the vicinity of the mitral annulus, such that the radial protrusion fills any gaps between outside of the frame and the walls of the left atrium in the vicinity of the mitral annulus.

In some applications, a size of the ring is smaller than a size of the subject's native mitral annulus.

In some applications, the radial protrusion is configured to generate tissue ingrowth to the radial protrusion from walls of the left atrium at least in the vicinity of the subject's native mitral annulus to the frame.

In some applications, the radial protrusion includes a flap.

In some applications, the radial protrusion includes a torus-shaped radial protrusion.

In some applications, the radial protrusion has an elliptical shape.

In some applications, the radial protrusion is configured to extend radially outwardly from the frame around more than 70 percent of a circumference of the frame.

In some applications, the docking element further includes a skirt that covers a portion of the frame extending from the ring to a height of at least 5 mm from the ring on the frame, and the bridging material includes a portion of the skirt.

In some applications, the radial protrusion is configured to generate tissue ingrowth to the radial protrusion from walls of the left atrium at least in the vicinity of the subject's native mitral annulus to the skirt.

In some applications, the radial protrusion is configured to self-expand.

In some applications, the radial protrusion includes a coil that is covered with a covering material.

In some applications, the frame includes struts that are joined to each other at junctions, such as to define cells, and in a vicinity of the junctions at least some of the struts are shaped to define undulating and/or zigzagging regions.

In some applications, by being shaped to define undulating and/or zigzagging regions, the effective strut length of the struts is elongated in the vicinity of the junctions, such as to spread strain that occurs in the vicinity of the junctions over a larger effective length than if the struts were not shaped to define the undulating and/or zigzagging regions.

In some applications, the frame includes struts that are joined to each other at junctions, such as to define cells, and at least some of the struts are arranged to define sets of two or more parallel struts between junctions, each of the struts in the set having a width of less than 0.4 mm.

In some applications, the sets of struts are disposed such that the adjacent long edges of each of the struts is disposed within 1 mm of each other when the frame is in a non-constrained configuration.

The present disclosure additionally provides a method for treating a subject with a diseased mitral valve, the method including:
inserting a docking element into a left atrium of the subject, the docking element including a ring, a frame extending from the ring, and a radial protrusion configured to extend radially outwardly from the frame;
deploying the docking element within the subject's left atrium, such that:
   no portion of the docking element extends through the subject's mitral valve,
   the radial protrusion is disposed in a vicinity of a native mitral annulus of the subject, such as to generate tissue ingrowth to the radial protrusion from walls of the left atrium at least in the vicinity of the subject's native mitral annulus,
   the frame anchors the docking element within the left atrium, prior to the tissue ingrowth to the radial protrusion occurring, by the frame expanding against walls of the left atrium and a roof of the left atrium, and
   a bridging material is disposed between the radial protrusion and the ring, the bridging material being configured to form a seal between the radial protrusion and the ring; and
subsequent to ingrowth of the tissue from walls of the left atrium to the radial protrusion having occurred:
   inserting a prosthetic mitral valve apparatus to inside the ring; and
   causing the prosthetic mitral valve apparatus to radially expand against the ring, such that the prosthetic mitral valve apparatus is anchored within the ring.

The present invention will be more fully understood from the following detailed description of embodiments thereof, taken together with the drawings, in which:

### BRIEF DESCRIPTION OF THE DRAWINGS

Figs. 1A, 1B, and 1C are schematic illustrations of a docking element configured to facilitate anchoring of prosthetic mitral valve apparatus to a subject's mitral valve, in accordance with some applications of the present invention;
Figs. 2A and 2B are schematic illustrations of a frame of a docking element that includes struts are shaped to have springy, undulating and/or zigzagging regions, in accordance with some applications of the present invention;
Fig. 3 is a schematic illustration of a frame of a docking element that includes struts are arranged in pairs of parallel struts, in accordance with some applications of the present invention; and
Figs. 4A and 4B are a schematic illustrations of fabric of a radial protrusion of a docking element and/or the fabric of a skirt of a docking element, in accordance with some applications of the present invention.

### DETAILED DESCRIPTION OF EMBODIMENTS

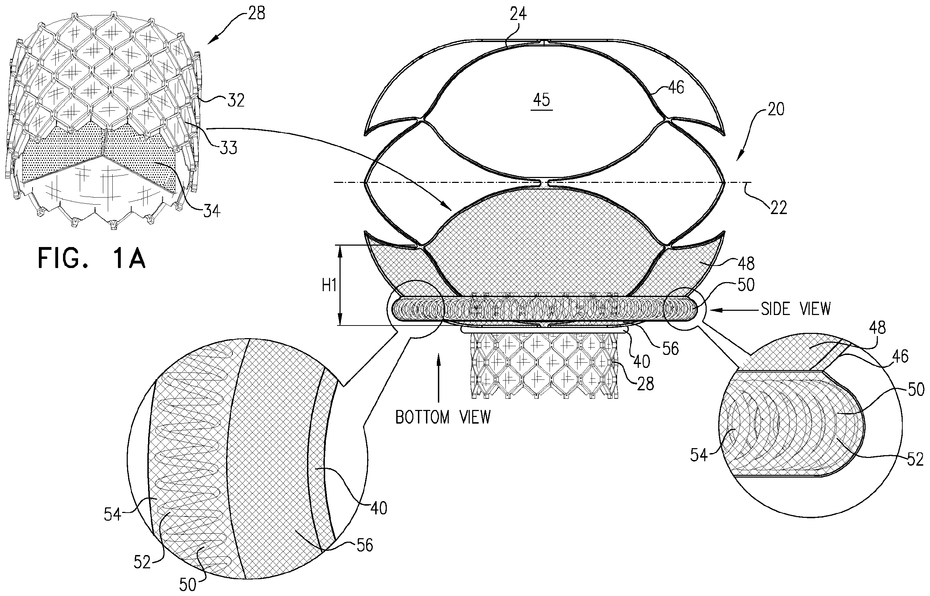

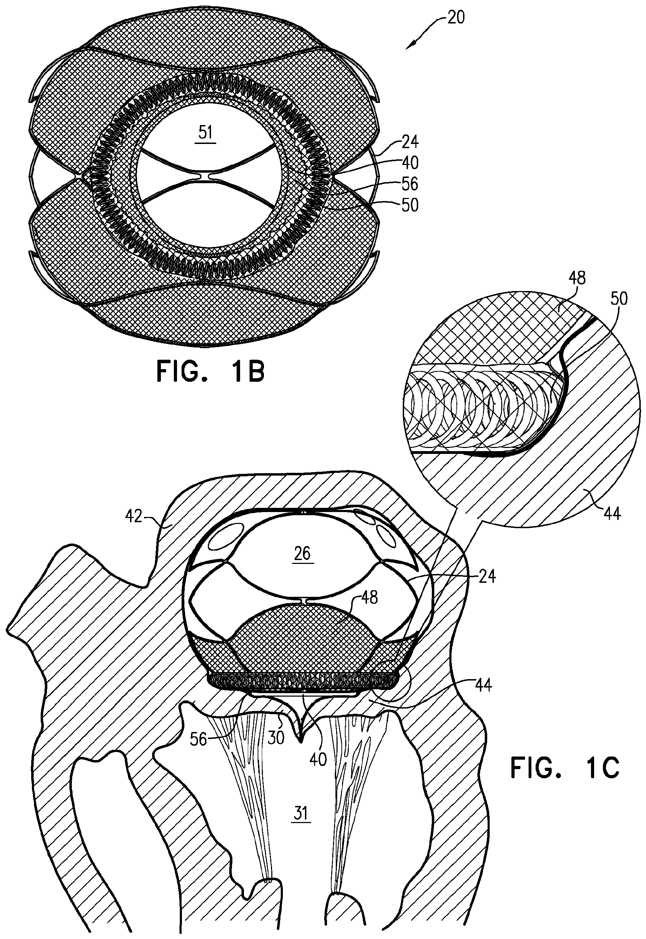

Reference is now made to Figs. 1A, 1B, and 1C, which are schematic illustrations of a docking element 20, in accordance with some applications of the present invention. Fig. 1A shows a side view of the docking element, and Fig. 1B shows a view of the docking element that is rotated through 90 degrees around a longitudinal axis 22 of a frame 24 of the docking element, relative to the view shown in Fig. 1A. Docking element is typically configured to facilitate anchoring of a prosthetic mitral valve apparatus 28 (shown in Fig. 1A) to a subject's mitral valve. Fig. 1C shows the docking element deployed inside a subject's left atrium 26, prior to the prosthetic mitral valve apparatus having been placed inside the docking element. As shown, in this state, the native mitral valve leaflets 30 are still in place. The docking element is disposed, in its entirety, within the left atrium 26, and does not protrude into left ventricle 31.

Typically, the prosthetic mitral valve apparatus is a stented valve that comprises a stented frame 32 that is configured to support prosthetic valve leaflets 34. Typically, the stented frame is covered with a covering material 33 (e.g., a fabric such as PET, PTFE, and/or nylon). The prosthetic mitral valve apparatus is typically implanted within the native mitral valve of a subject with a diseased native valve, and the prosthetic valve leaflets function such as to replace the functioning of the native valve leaflets. Typically, the prosthetic valve leaflets are configured to act as a one-way valve, whereby in their open positions with respect to one another the leaflets allow flow to pass through the prosthetic valve apparatus from the inlet (on the atrial side) to the outlet (on the ventricular side), whereas reverse flow is prevented due to collapsible slack portions of the valve leaflets collapsing inwardly to block the reverse flow.

Typically, docking element 20 is implanted within the left atrium more than one week, or more than one month, before the implantation of the prosthetic mitral valve apparatus. Subsequent to the implantation of the docking element, and before the implantation of the prosthetic mitral valve apparatus, the anchoring of the docking element is typically strengthened by virtue of tissue ingrowth that occurs around the docking element, e.g., as described hereinbelow. Typically, no portion of the docking element extends through the subject's native mitral valve. Further typically, by virtue of the fact that no portion of the docking element extends through the subject's native mitral valve, the native mitral valve leaflets are able to continue functioning in their normal manner subsequent to the implantation of the docking element, and prior to the implantation of the prosthetic mitral valve apparatus.

For some applications, the docking element includes frame 24 and a ring 40 coupled to the frame. Ring 40 is described in further detail hereinbelow. Typically, in its deployed state inside the left atrium, the ring is disposed transversely with respect to the frame, such that a plane defined by the ring is substantially parallel to the longitudinal axis of the frame.

For some applications, the docking element is placed into left atrium 26, via interatrial septum 42 (shown in Fig. 1C), by advancing the docking element, along its longitudinal axis, in a lateral direction with respect to the subject's left atrium. Subsequently, substantially without rotating the longitudinal axis of the docking element, the docking element is deployed within the subject's left atrium, such that ring 40 is disposed in the vicinity of mitral annulus 44 (e.g., at the mitral annulus, and/or slightly above (e.g., as described hereinabove)), and is disposed transversely with respect to the frame. (It is noted that, in some cases, the frame may be rotated around the longitudinal axis, and/or there may be a small amount of rotation of the longitudinal axis itself, in order to align the ring with the native mitral annulus.) Thus, even in the deployed state of the docking element, the longitudinal axis of the frame is substantially parallel to a plane defined by the subject's mitral annulus. By virtue of the docking element deploying in this manner, the longitudinal axis of the docking element does not need to be substantially rotated with respect to the left atrium between being transseptally inserted into the left atrium, and being deployed within the left atrium. By contrast, if the ring were disposed at a longitudinal end of the frame such that the plane defined by the ring was substantially perpendicular to the longitudinal axis of the frame, this would make it challenging to insert the docking element transseptally. This is because the longitudinal axis of the docking element would need to be rotated (approximately 90 degrees relative to the interatrial septum) and released from the delivery device within the relatively small volume that is defined by the left atrium.

For some applications, the ring is configured to be disposed at a height that is level with, slightly above, or slightly below the mitral annulus, and the outer perimeter of the ring is smaller than the perimeter defined by the mitral annulus, such that the ring "floats" above the native valve leaflets 30. Such applications of the present invention are described in further detail hereinbelow.

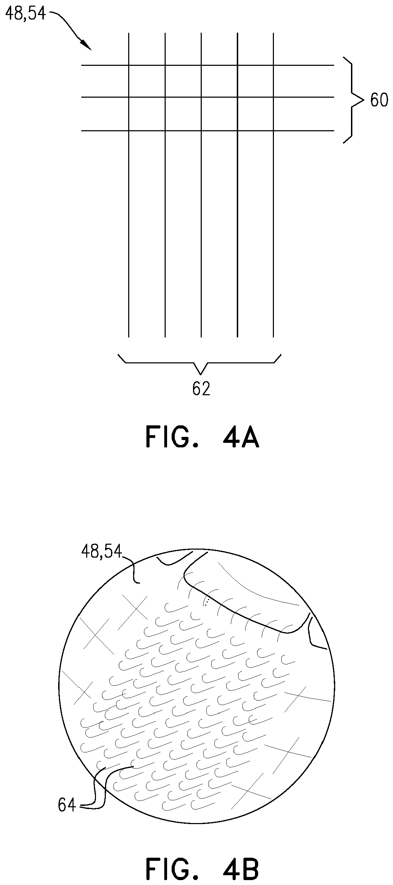

Typically, frame 24 is made of a self-expandable, shape memory material (e.g., a shape memory alloy, such as nitinol), that is cut and shape set such as to define a plurality of cells 45 defined by struts 46 of the shape memory material. Alternatively or additionally, the frame is assembled from wires that are made of a self-expandable, shape memory material (e.g., a shape memory alloy, such as nitinol). Typically, a portion of the frame that is closest to the ring is covered in a skirt 48 of a material that facilitates tissue ingrowth to the frame. For example, the skirt may be made of a fabric such as PET, PTFE, and/or nylon, and may be coupled to the outside of the frame and/or the ring using stitches. Typically, the portion of the frame that is configured to be disposed in the vicinity of the mitral annulus ( e.g., at the mitral annulus, and/or slightly above (e.g., within 15 mm of) the mitral annulus)) is covered with the skirt. For some applications, the skirt is configured to be disposed from between 1 mm and 2 mm above the mitral annulus (or from the height of the annulus) until a height of between 15 mm and 20 mm above the mitral annulus. For some applications, tissue-ingrowth elements are coupled to the skirt to encourage tissue ingrowth, e.g., as described hereinbelow with reference to Fig. 4B. For example, such tissue-ingrowth elements may include hooks or loops (e.g., similar to those used in hook-and-loop fasteners), barbs, clips, pins, etc. For some applications, the skirt extends from the ring to a height H1 on the frame of at least 5 mm (e.g., at least 10 mm), and/or less than 25 mm (e.g., less than 20 mm), e.g., between 5 and 25 mm, or between 10 and 20 mm. For some applications (not shown), from where the skirt terminates (i.e., from the upper end of the skirt), struts of the frame are covered in sleeves of a material that is typically the same as the material from which skirt 48 is made. The sleeves of material are configured to encourage tissue ingrowth to the struts of the frame. Typically, at junctions between struts, the sleeves are stitched together. For some applications, the docking element is deployed in the left atrium, such that frame 24 extends to the roof of the left atrium (as shown in Fig. 1C).

For some applications, the docking element includes a radial protrusion 50 that extends radially from the frame, typically above ring 40 and/or level with ring 40. For example, the radial protrusion may be shaped as a flap and/or a torus that is disposed around the circumference of the frame. For some applications, the radial protrusion is configured to conform to the shape of the walls of the left atrium in the vicinity of the mitral annulus, such that the radial protrusion fills any gaps between the outside of the frame and walls of the left atrium in the vicinity of the mitral annulus. Typically, the radial protrusion is configured to contact the inner walls of the left atrium, and to promote tissue ingrowth from the inner walls of the left atrium to the protrusion. Thus, the docking element typically becomes anchored to the inner walls of the left atrium by virtue of the tissue ingrowth. For some applications, the protrusion is configured to act as a bridge for the tissue ingrowth, with the protrusion being configured to encourage tissue ingrowth from the inner walls of the left atrium, across the protrusion, and into skirt 48 and/or into frame 24. Typically, the protrusion is configured to contact the inner walls of the left atrium in the vicinity of the mitral annulus, e.g., at the height of the mitral annulus, and/or from between 1 mm and 2 mm above the mitral annulus until a height of between 15 mm and 20 mm above the mitral annulus.

Typically, the outer perimeter of the ring is smaller than the size of the inner perimeter of radial protrusion 50. Further typically, there is a bridging material 56 (e.g., a fabric) that bridges and forms a seal between the ring and the radial protrusion. Typically, the bridging material 56 that bridges between the ring and the radial protrusion is a portion of skirt 48. In accordance with the above description, for some applications, the docking element includes a combination of three components: 1) protrusion 50, which is configured to become anchored to the tissue in the vicinity of the mitral annulus, via tissue ingrowth, 2) ring 40, to which the prosthetic mitral valve apparatus is configured to become anchored, and 3) bridging material 56 that bridges and forms a seal between these two elements. For some applications, protrusion 50 protrudes directly from the outside of ring 40. For some applications, the protrusion is elliptical in shape, and a first portion of the protrusion (e.g., the sides of the ellipse that encompass the minor axis) protrudes directly from ring 40, and a second portion of the protrusion (e.g., the sides of the ellipse that encompass the major axis) is coupled to the ring via bridging material 56.

For some applications, radial protrusion 50 is self-expandable and includes a coiled spring 52 (which is typically made of a self-expandable metal or alloy, such a nitinol) covered with a fabric 54 (such as PET, PTFE, and/or nylon) that is configured to promote tissue ingrowth. In accordance with respective embodiments, coiled spring 52 is a round coil, a triangular coil, or a differently shaped coil. The coiled spring is configured to cause the self-expandable protrusion to extend radially outwardly from the outer surface of the frame, such that the protrusion conforms with the anatomy of the left atrium in regions in which the frame itself is not compliant enough to do so. In this manner, the self-expandable protrusion fills the gaps between the outer surface of the frame and the inner walls of the left atrium.

For some applications, radial protrusion 50 is used in conjunction with other devices that are configured to be placed within a chamber of the subject's heart, such as a mitral annulus repair device, e.g., as described in US 2020/0093599 to Benichou *mutatis mutandis.*

For some applications, radial protrusion 50 comprises a material that is filled with a substance (e.g., a hydrogel) that is configured to expand once in contact with blood. For some applications, the protrusion is not self-expandable, but is rather an inflatable protrusion. For example, the protrusion may include an inflatable torus that is inflated with an inflation substance (e.g., an epoxy material). For some applications, the protrusion is configured as a brush strip. Alternatively or additionally, the protrusion is generally similar to that shown in Figs. 1A-C, but rather than including spring 52, a wire (e.g., configured in a ring or in a zigzag configuration) extends radially outwardly from the frame and is configured to support fabric 54, such that the fabric extends approximately perpendicularly from the outer surface of the frame. Typically, radial protrusion 50 extends radially outwardly from the outer surface of frame 24 around more than 70 percent (e.g., more than 90 percent) of the circumference of the frame. Further typically, protrusion 50 extends radially outwardly from the outer surface of frame 24 around the full circumference of the frame. For some applications, the radial protrusion is disposed on the frame at a height that is level with ring 40. Alternatively or additionally, the protrusion protrudes from the frame at a height above the ring of between 1 mm 20 mm (e.g., between 2 mm and 15 mm).

As described hereinabove, typically, docking element 20 is configured to facilitate anchoring of prosthetic mitral valve apparatus 28 to the subject's mitral valve. The docking element typically includes (a) ring 40 (within which the prosthetic valve apparatus is anchored), and (b) a portion of the frame that is covered with skirt 48, such as to encourage tissue ingrowth from the atrial walls in the vicinity of (e.g., at the height of or slightly above (e.g., within 15 mm of)) the mitral annulus to that portion. The portion of the frame that is covered with skirt 48 is typically configured to be sized such that, when the docking element is in a deployed configuration, this portion of the frame conforms with the size of the left atrium. Typically, the size of the ring (e.g., the outer perimeter of the ring) is smaller than this portion of the frame. Further typically, a portion of skirt 48 bridges and seals between the ring and this portion of the frame. In this manner, for some applications, the docking element occupies and seals some of the area defined by the native mitral annulus. Thus, the ring acts as an artificial mitral annulus that is smaller than the native mitral annulus. Typically, the inner diameter of ring 40 is more than 20 mm. For some applications, the inner diameter of ring 40 is smaller than the inner diameter of native mitral annulus 44. For example, the inner diameter of ring 40 may be less than 30 mm, e.g., less than 28 mm.

For some applications, the docking element includes frame 24, ring 40, skirt 48 and radial protrusion 50. The radial protrusion is configured to extend to the walls of the left atrium in the vicinity of (e.g., at the height of or slightly above (e.g., within 15 mm of)) the native mitral annulus, and to encourage tissue ingrowth from the subject's left atrium to the docking element, e.g., tissue ingrowth to the radial protrusion itself, and/or tissue ingrowth via the radial protrusion to the frame and/or the skirt. A portion of skirt 48 acts as bridging material 56 and bridges and seals between the radial protrusion and the ring, such that the bridging material occupies and seals some of the area defined by the native mitral annulus. Thus, the ring acts as an artificial mitral annulus that is smaller than the native mitral annulus.

In accordance with the above, for some applications, the size of the prosthetic mitral valve is smaller than that of the native mitral valve. For example, prosthetic valve leaflets 34 of the prosthetic mitral valve apparatus may span a diameter that is less than the measured diameter of the native mitral annulus (the diameter of the native mitral annulus typically being measured using a mitral measuring ring, and/or using imaging methods, such as ultrasound). Or, since the native mitral annulus is typically elliptical, the maximum diameter that is spanned by the leaflets may be less than the major axis of the ellipse defined by the native mitral annulus.

For some applications, one or more advantages of the prosthetic valve being sized in this manner, relative to if the prosthetic valve were to be larger, may include: the prosthetic valve apparatus having a lower crimped profile (and therefore the use of a delivery device having a smaller diameter), there being less foreign matter inside the subject's heart, lower forces being exerted on the prosthetic valve leaflets (and therefore improved durability), better anchoring of the prosthetic valve apparatus, less interference with the native anatomy, and/or better preservation of a clear left ventricular outflow tract. Typically, smaller sized leaflets allow the frame of the prosthetic mitral valve apparatus to be shorter, thereby reducing obstruction of the left ventricular outflow tract, *ceteris paribus.* Alternatively or additionally, prosthetic mitral valve apparatus having prosthetic valve leaflets spanning a diameter that is less than that of the native mitral annulus may be used for a different reason.

Referring to Fig. 1B, frame 24 typically defines a cell 51 that is configured to be disposed at the side of the frame that is placed in the vicinity of the mitral annulus. For some applications, cell 51 defines an open area of more than 3.0 square cm (e.g., more than 4.0 square cm), and/or less than 6.0 square cm (e.g., less than 5.5 square cm), e.g., 3.0-6.0 square cm (or 4.0-5.5 square cm). Typically, ring 40 is disposed inside cell 51. Further typically, skirt 48 covers any space between struts that define cell 51 and ring 40, such as to seal this space. For some applications, the skirt additional covers spaces (or portions thereof) defined by additional cells. Typically, the ring is coupled to the struts that define cell 51 via skirt 48. For example, skirt 48 may be stitched to both the struts that define cell 51, and to ring 40. For some applications, ring 40 is directly coupled to the struts that define cell 51 (not shown). For some applications, a portion of skirt 48 additionally acts as bridging material 56, bridging between ring 40 and radial protrusion 50.

Typically, prior to the tissue ingrowth having occurred, the docking element is anchored in place within the left atrium by virtue of the frame expanding such as to contact the walls and the roof of the left atrium. For some applications, the frame is configured to only apply a relatively low pressure to the inner walls of the left atrium, such that the frame conforms to the shape of the left atrium, rather than forcing the atrium to deform. For some applications, in this manner, the frame allows the natural compliance of the inner walls of the left atrium to be substantially maintained. For example, the frame may be configured such that 1N of force is required for a reduction in diameter of the frame of 10 mm. For some applications, each of cells 45 of the frame defines an open area of more than 3.0 square cm (e.g., more than 4.0 square cm), and/or less than 6.0 square cm (e.g., less than 5.5 square cm), e.g., 3.0-6.0 square cm (or 4.0-5.5 square cm). For some applications, the cells are disposed such that the open portions of the cells are disposed at junctions of the pulmonary veins with the left atrium. Typically, skirt 48 does not extend to a height upon the frame that is disposed junctions of the pulmonary veins with the left atrium. In this manner the frame does not interfere with blood flow from the pulmonary veins to the left atrium. It is noted that frame 24 is typically configured such that contact between the frame and left atrium is spread substantially evenly over the walls of the left atrium and roof of the left atrium. It is further noted that, typically, the frame does not include any additional anchoring portions for anchoring to body portions (such as the left atrial appendage and/or pulmonary veins), other than the walls and the roof of the left atrium.

Typically, docking element 20 becomes anchored to the subject's heart by virtue of (a) tissue ingrowth to the portion of the frame that is disposed in the vicinity of the mitral annulus (e.g., at the mitral annulus, and/or slightly above the mitral annulus (e.g., as described hereinabove)) and is covered with skirt 48, (b) tissue ingrowth from the left atrium walls to the struts 46 of the frame, (c) tissue ingrowth to radial protrusion 50, and/or (d) tissue ingrowth to the frame and/or the skirt via the radial protrusion. Typically, prior to the tissue ingrowth having occurred, the docking element is anchored in place within the left atrium by virtue of the frame expanding such as to contact the walls and the roof of the left atrium.

Typically, the docking element is configured such that, by the time that the prosthetic mitral valve apparatus is implanted, the anchoring of the docking element within the left atrium is primarily via the tissue ingrowth to the docking element. Typically, the mitral annulus is able to bear a higher load than other portions of the left atrial walls. Therefore, for some applications, the docking element is configured such that, when the prosthetic mitral valve apparatus is implanted, the anchoring of the docking element within the left atrium is primarily via the tissue ingrowth from the atrial walls in the vicinity of (e.g., at the height of, or slightly above (e.g., within 15 mm of)) the mitral annulus to the docking element. For example, by virtue of the docking element including skirt 48 (which is configured to be disposed in the vicinity of the mitral annulus), the docking element may be configured to encourage greater tissue ingrowth in the vicinity of the mitral annulus than at other portions of the frame. Alternatively or additionally, by virtue of the docking element including radial protrusion 50 (which is configured to be disposed in the vicinity of the mitral annulus), the docking element is configured to encourage greater tissue ingrowth in the vicinity of the mitral annulus than at other portions of the frame.

The above-described, two-stage implantation procedure is somewhat analogous to a valve-in-valve procedure, whereby a new prosthetic valve is implanted inside a previously-implanted prosthetic valve. The previously-implanted prosthetic valve is typically initially anchored to the native mitral valve via suturing, but subsequently becomes anchored by virtue of tissue ingrowth to the prosthetic valve. In such cases, the new valve typically becomes anchored within the previously-implanted valve, and the previously-implanted valve is strongly anchored to the native mitral valve, by virtue of tissue ingrowth with respect to the previously-implanted valve, as well as mechanical force exerted upon the heart by the previously-implanted prosthetic valve. Similarly, in accordance with some applications of the present invention, initially, docking element 20 is implanted and is allowed to become anchored to the subject's heart by virtue of tissue ingrowth with respect to the docking element, as well as mechanical force exerted upon the heart by the docking element. Subsequently, once the docking element is anchored within the subject's heart, prosthetic mitral valve apparatus is anchored to the docking element (and typically to ring 40 of the docking element). For some applications, stented frame 32 of prosthetic mitral valve apparatus 28 has an hourglass-shaped outer profile, that is configured to facilitate anchoring of the frame to ring 40 of docking element 20.

In accordance with respective applications of the present invention, docking element 20 and/or prosthetic mitral valve apparatus 28 is delivered to the subject's heart via the femoral vein, transapically, transseptally, and/or transaortically. For some applications, by delivering docking element 20 and prosthetic mitral valve apparatus 28 in separate delivery steps, the size of the delivery device(s) that is/are used to deliver docking element 20 and prosthetic mitral valve apparatus 28 may be smaller than if the prosthetic mitral valve apparatus was to be delivered together with the docking element, *ceteris paribus.* For some applications, reducing the size of the delivery device(s) that is/are required, in the above-described manner, facilitates transseptal insertion of the docking element and/or the prosthetic mitral valve apparatus.

For some applications, docking element 20 and prosthetic mitral valve apparatus 28 are implanted above the native mitral valve in the same procedure as each other, with the docking element typically being implanted prior to the prosthetic mitral valve apparatus. For some such applications, when the prosthetic mitral valve apparatus is initially implanted, the prosthetic mitral valve leaflets 34 are held open, for example, using sutures. Typically, the prosthetic mitral valve leaflets are maintained in the open state (e.g., using the sutures) for a period of more than one week, or more than one month, during which period the docking element becomes anchored to the subject's heart by virtue of tissue ingrowth, in accordance with the techniques described hereinabove. In this manner, during the period in which the docking element is becoming anchored to the subject's heart by virtue of tissue ingrowth, the docking element is not required to bear the load of blood impacting the prosthetic valve leaflets. Subsequently, the element that is keeping the prosthetic valve leaflets open (e.g., the sutures) is removed, such that the prosthetic valve leaflets begin functioning.

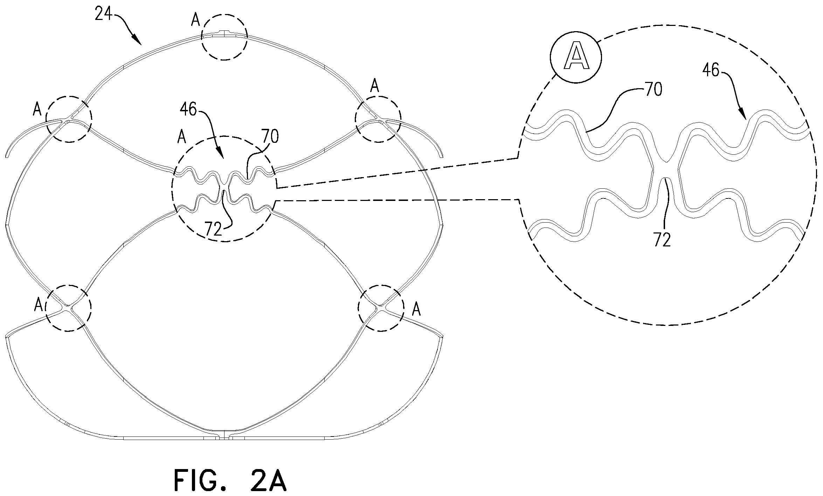

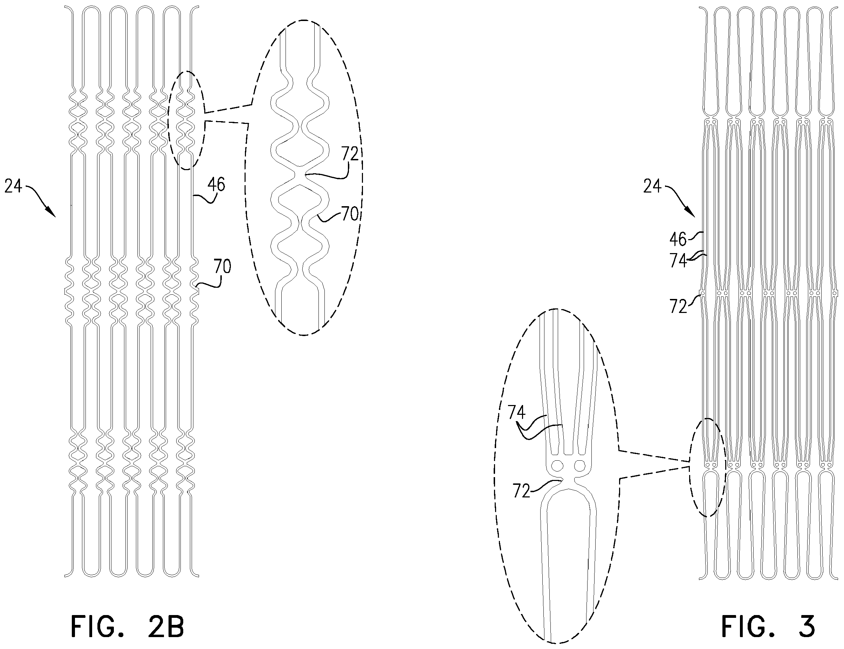

Reference is now made to Figs. 2A and 2B, which are schematic illustrations of frame 24 of docking element 20, the frame including struts 46 that are shaped to have springy, undulating and/or zigzagging regions 70, in accordance with some applications of the present invention, in accordance with some applications of the present invention. Fig. 2A is a schematic illustration of frame 24 in its non-radially constrained configuration, and in the absence of other portions of the docking element. Fig. 2B is a schematic illustration of a flattened profile of the frame, which depicts (for illustrative purposes) how the frame would appear if, prior to shape setting the frame, a longitudinal incision was to be made along the length of the frame at a given circumferential location of the frame, and the frame were to then be laid out flat upon a surface. Frame 24 as shown in Figs. 2A-B is generally similar to frame 24 as described hereinabove (for example, with reference to Figs. 1A-C), except for the following differences.

As described hereinabove, typically, frame 24 is configured to conform with the shape of the atrial walls and roof. As such, the frame typically expands and contracts over the course of the subject's cardiac cycle. As the frame expands and contract, the angles between struts 46 of the frame changes. Typically, most of the stress and strain that the struts undergo as the angles between the struts change occurs at the junctions between the struts. As shown in Figs. 2A-B, for some applications, in the vicinity of junctions 72 between struts 46 of frame 24, the struts are shaped to have springy, undulating and/or zigzagging regions 70. For example, the springy, undulating and/or zigzagging regions may be within between 1 mm and 20 mm (e.g., within between 2 mm and 15 mm) of the junctions (as measured along the longitudinal axes of the straight portions of the struts). In this manner, the effective strut length is elongated in the vicinity of the junctions, which spreads the stress and strain that occurs in the vicinity of the junctions over a larger effective length. (It is noted that, in Fig. 2A, each of the regions marked with the letter A next to a dashed circle typically includes undulating and/or zigzagging strut regions, but only one such region is depicted for illustrative purposes.)

Reference is now made to Fig. 3, which is a schematic illustration of frame 24 of docking element 20, the frame including struts 46 that are arranged in pairs of parallel struts, in accordance with some applications of the present invention. Fig. 3 is a schematic illustration of a flattened profile of frame 24, which depicts (for illustrative purposes) how the frame would appear if, prior to shape setting the frame, a longitudinal incision was to be made along the length of the frame at a given circumferential location of the frame, and the frame were to then be laid out flat upon a surface. Frame 24 as shown in Fig. 3 is generally similar to frame 24 as described hereinabove (for example, with reference to Figs. 1A-C), except for the following differences.

As shown in Fig. 3, for some applications, the struts are relatively thin, in order to reduce strain that develops in the struts as a result of bending. For example, the struts may have widths of less than 0.4 mm, e.g., less than 0.3 mm. The struts being relatively thin can cause the struts to have relatively low rigidity. Therefore, for some applications, within at least a portion of frame 24, the struts are arranged in pairs 74 of parallel struts, in order to provide greater rigidity to the struts. Typically, the pairs 74 of struts are disposed such that the adjacent long edges of each of the struts is disposed within 1 mm of each other (e.g., within 0.6 mm of each other), when the frame is disposed in a non-constrained configuration. For some applications, more than two struts (i.e., three or more struts) are arranged in parallel with each other. Typically, the parallel struts extend from a single junction 72, and lie in the same plane as each other and in the same plane as the junction, such that the struts are configured not to rotate with respect to each other. For some applications (not shown), each of the individual struts in the pair is in the form of a cable and/or a coiled spring. For some applications, individual struts of frame 24 (i.e., struts that are not arranged in parallel with other struts (e.g., struts of frame 24 as shown in any one of Figs. 1A-C, or 2A-B)) are in the form of a cable and/or coiled springs.

Reference is now made to Fig. 4A, which is a schematic illustration of fabric 54 of radial protrusion 50 and/or the fabric of skirt 48, the fabric being woven, in accordance with some applications of the present invention. For some applications, the woven fabric is made of combination of elastic wires 60 (made of a metal or alloy, such as nitinol) and a polymeric material 62 (such as PET, PTFE, and/or nylon). Typically, the density and direction of the each of the materials within the weave is such as to provide the fabric with the desired characteristics of the portion of the docking element within which it is used.

Reference is now made to Fig. 4B, which is a schematic illustration of fabric 54 of radial protrusion 50 and/or the fabric of skirt 48, in accordance with some applications of the present invention. For some applications, tissue-ingrowth elements 64 are coupled to the fabric layer to encourage tissue ingrowth. For example, such tissue-ingrowth elements may include hooks or loops (e.g., similar to those used in hook-and-loop fasteners), barbs, clips, pins, etc. The tissue-ingrowth elements are configured to rub against the walls of the left atrium and to encourage tissue growth into the fabric of the fabric layer. For some applications, the tissue-ingrowth elements include fabric sheets, to which fibers (e.g., hook shaped fibers, as shown) are coupled in a perpendicular arrangement with respect to the fabric sheets. For some applications, the fibers are coupled directly to fabric 54 of radial protrusion 50 and/or the fabric of skirt 48. The fibers are configured to rub against the walls of the left atrium and to encourage tissue growth into the fibers and the fabric sheets. For some applications, the tissue-ingrowth elements define a 3D structure (e.g., as described hereinabove) that is configured to increase the contact area between the tissue and the frame and to serve as a scaffold for tissue to grow into.

For some applications, a portion of docking element is biodegradable. For example, at least a portion of frame 24 may be biodegradable. Typically, even for such applications, skirt 48, ring 40, and/or radial protrusion 50 are not biodegradable. For some such applications, after a given time period (by which time the tissue ingrowth has occurred), the frame biodegrades leaving only ring 40, skirt 48, and/or protrusion 50 in place. Alternatively or additionally, skirt 48 and/or radial protrusion 50 is sewn to the frame via biodegradable sutures. Typically, after tissue ingrowth has occurred with respect to the skirt and/or the radial protrusion, the sutures biodegrade and the frame is removed from the subject's left atrium. For some such applications, the frame is covered with an anti-tissue-ingrowth coating, such that tissue ingrowth does not occur with respect to the frame and the frame can be readily removed.

For some applications, the docking element includes a left-atrial appendage anchor, e.g., as described in WO 18/178966, which is incorporated herein by reference. Alternatively or additionally, the docking element includes a left-atrial appendage closure portion that is configured to close the ostium of the left-atrial appendage. For some applications, the docking element includes an anchor and/or a closure portion that is configured to become anchored to or to close the fossa ovalis.

For some applications, the apparatus and methods described herein are performed with respect to a tricuspid valve, and/or a different valve in a subject's body, *mutatis mutandis.*

For some applications, the apparatus and methods described herein are performed in conjunction with apparatus and methods described in:
US 16/498,065 to Benichou et al., entitled "Docking elements," which is the US national phase of International Patent Application No. PCT/IL2018/050229 to Benichou et al. (published as WO 18/178966), entitled "Docking elements," filed March 01, 2018, which claims priority from US Provisional Application 62/476,989 to Benichou, entitled "Docking element," filed March 27, 2017; and/or
US 2020/0093599 to Benichou et al., entitled "Docking elements," filed Sep. 24, 2019, which claims priority from US Provisional application 62/735,866 to Benichou et al., entitled "Docking elements," filed September 25, 2018.

It will be appreciated by persons skilled in the art that the present invention is not limited to what has been particularly shown and described hereinabove. Rather, the scope of the present invention is limited by the scope of the appended claims.

## Claims

1. Apparatus for treating a subject with a diseased mitral valve, the apparatus comprising:
a docking element (20) configured to be implanted within a left atrium of the subject such that no portion of the docking element (20) extends through the subject's mitral valve, the docking element (20) comprising:
a ring (40);
a frame (24) extending upwardly from the ring (40), the frame (24) being configured to anchor the docking element (20) within the left atrium, prior to the tissue ingrowth to the docking element (20) occurring, by the frame (24) expanding against at least one of walls of the left atrium and a roof of the left atrium;
a radial protrusion (50) configured to extend radially outwardly from the frame (24), to be disposed in a vicinity of the subject's native mitral annulus, and to generate tissue ingrowth to the radial protrusion (50) from walls of the left atrium at least in the vicinity of the subject's native mitral annulus; and
a bridging material (56) disposed between radial protrusion (50) and the ring (40), the bridging material (56) forming a seal between the radial protrusion (50) and the ring (40); and
a prosthetic mitral valve apparatus configured:
subsequent to the ingrowth from walls of the left atrium to the radial protrusion (50) having occurred, to be placed at least partially inside the docking element (20), and
to become anchored to the docking element (20), at least partially by radially expanding against the ring (40).

2. The apparatus according to claim 1, wherein the radial protrusion (50) is configured to extend radially outwardly from the frame (24) at a height that is level with the ring (40).

3. The apparatus according to claim 1, wherein the radial protrusion (50) is configured to extend radially outwardly from the frame (24) at a height that is between 1 mm and 20 mm above the ring (40).

4. The apparatus according to claim 1, wherein the radial protrusion (50) is configured to conform to the shape of the walls of the left atrium in the vicinity of the mitral annulus, such that the radial protrusion (50) fills any gaps between outside of the frame (24) and the walls of the left atrium in the vicinity of the mitral annulus.

5. The apparatus according to claim 1, wherein a size of the ring (40) is smaller than a size of the subject's native mitral annulus.

6. The apparatus according to claim 1, wherein the radial protrusion (50) is configured to generate tissue ingrowth to the radial protrusion (50) from walls of the left atrium at least in the vicinity of the subject's native mitral annulus to the frame (24).

7. The apparatus according to claim 1, wherein the radial protrusion (50) comprises a flap, or wherein the radial protrusion (50) comprises a torus-shaped radial protrusion (50), or wherein the radial protrusion (50) has an elliptical shape.

8. The apparatus according to claim 1, wherein the radial protrusion (50) is configured to extend radially outwardly from the frame (24) around more than 70 percent of a circumference of the frame (24).

9. The apparatus according to any one of claims 1-8, wherein the docking element (20) further comprises a skirt that covers a portion of the frame (24) extending from the ring (40) to a height of at least 5 mm from the ring (40) on the frame (24), and wherein the bridging material (56) comprises a portion of the skirt.

10. The apparatus according to claim 9, wherein the radial protrusion (50) is configured to generate tissue ingrowth to the radial protrusion (50) from walls of the left atrium at least in the vicinity of the subject's native mitral annulus to the skirt.

11. The apparatus according to any one of claims 1-8, wherein the radial protrusion (50) is configured to self-expand.

12. The apparatus according to claim 11, wherein the radial protrusion (50) comprises a coil (52) that is covered with a covering material (54).

13. The apparatus according to any one of claims 1-8, wherein the frame (24) comprises struts that are joined to each other at junctions (72), such as to define cells, and wherein in a vicinity of the junctions at least some of the struts are shaped to define undulating and/or zigzagging regions (70).

14. The apparatus according to claim 13, wherein, by being shaped to define undulating and/or zigzagging regions (70), the effective strut length of the struts is elongated in the vicinity of the junctions, such as to spread strain that occurs in the vicinity of the junctions over a larger effective length than if the struts were not shaped to define the undulating and/or zigzagging regions.

15. The apparatus according to any one of claims 1-8, wherein frame (24) comprises struts that are joined to each other at junctions (72), such as to define cells, and wherein at least some of the struts are arranged to define sets of two or more parallel struts (74) between junctions, each of the struts in the set having a width of less than 0.4 mm.

## Patentansprüche

1. Vorrichtung zur Behandlung eines Patienten mit einer erkrankten Mitralklappe, wobei die Vorrichtung Folgendes umfasst:
ein Andockelement (20), das dazu ausgestaltet ist, in einem linken Vorhof des Patienten implantiert zu werden, so dass sich kein Abschnitt des Andockelements (20) durch die Mitralklappe des Patienten erstreckt, wobei das Andockelement (20) Folgendes umfasst:
einen Ring (40),
einen Rahmen (24), der sich von dem Ring (40) nach oben erstreckt, wobei der Rahmen (24) dazu ausgestaltet ist, das Andockelement (20) in dem linken Vorhof zu verankern, bevor das Hineinwachsen von Gewebe in das Andockelement (20) erfolgt, indem der Rahmen (24) gegen mindestens eine der Wände des linken Vorhofs und eine Decke des linken Vorhofs expandiert,
einen radialen Vorsprung (50), der dazu ausgestaltet ist, sich von dem Rahmen (24) radial nach außen zu erstrecken, in einer Nähe des nativen Mitralanulus des Patienten angeordnet zu sein und das Hineinwachsen von Gewebe von Wänden des linken Vorhofs zu dem radialen Vorsprung (50) mindestens in der Nähe des nativen Mitralanulus des Patienten zu erzeugen, und
ein Überbrückungsmaterial (56), das zwischen dem radialen Vorsprung (50) und dem Ring (40) angeordnet ist, wobei das Überbrückungsmaterial (56) eine Dichtung zwischen dem radialen Vorsprung (50) und dem Ring (40) bildet, und
eine Mitralklappenvorrichtungsprothese, die dazu ausgestaltet ist,
nach dem Erfolgen des Hineinwachsens von Wänden des linken Vorhofs zu dem radialen Vorsprung (50) mindestens teilweise in dem Andockelement (20) platziert zu sein, und
mindestens teilweise durch radiales Expandieren gegen den Ring (40) an dem Andockelement (20) verankert zu werden.

2. Vorrichtung nach Anspruch 1, wobei der radiale Vorsprung (50) dazu ausgestaltet ist, sich auf einer Höhe, die auf demselben Niveau wie der Ring (40) liegt, von dem Rahmen (24) radial nach außen zu erstrecken.

3. Vorrichtung nach Anspruch 1, wobei der radiale Vorsprung (50) dazu ausgestaltet ist, sich auf einer Höhe, die zwischen 1 mm und 20 mm über dem Ring (40) liegt, von dem Rahmen (24) radial nach außen zu erstrecken.

4. Vorrichtung nach Anspruch 1, wobei der radiale Vorsprung (50) dazu ausgestaltet ist, an die Form der Wände des linken Vorhofs in der Nähe des Mitralanulus angepasst zu sein, so dass der radiale Vorsprung (50) etwaige Lücken zwischen außerhalb des Rahmens (24) und den Wänden des linken Vorhofs in der Nähe des Mitralanulus füllt.

5. Vorrichtung nach Anspruch 1, wobei eine Größe des Rings (40) kleiner als eine Größe des nativen Mitralanulus des Patienten ist.

6. Vorrichtung nach Anspruch 1, wobei der radiale Vorsprung (50) dazu ausgestaltet ist, das Hineinwachsen von Gewebe zu dem radialen Vorsprung (50) von Wänden des linken Vorhofs mindestens in der Nähe des nativen Mitralanulus des Patienten zu dem Rahmen (24) zu erzeugen.

7. Vorrichtung nach Anspruch 1, wobei der radiale Vorsprung (50) eine Klappe umfasst oder wobei der radiale Vorsprung (50) einen torusförmigen radialen Vorsprung (50) umfasst oder wobei der radiale Vorsprung (50) eine elliptische Form hat.

8. Vorrichtung nach Anspruch 1, wobei der radiale Vorsprung (50) dazu ausgestaltet ist, sich von dem Rahmen (24) um mehr als 70 Prozent eines Umfangs des Rahmens (24) radial nach außen zu erstrecken.

9. Vorrichtung nach einem der Ansprüche 1 - 8, wobei das Andockelement (20) ferner eine Schürze umfasst, die einen Abschnitt des Rahmens (24) abdeckt, der sich von dem Ring (40) zu einer Höhe von mindestens 5 mm ab dem Ring (40) an dem Rahmen (24) erstreckt, und wobei das Überbrückungsmaterial (56) einen Abschnitt der Schürze umfasst.

10. Vorrichtung nach Anspruch 9, wobei der radiale Vorsprung (50) dazu ausgestaltet ist, das Hineinwachsen von Gewebe zu dem radialen Vorsprung (50) von Wänden des linken Vorhofs mindestens in der Nähe des nativen Mitralanulus des Patienten zu der Schürze zu erzeugen.

11. Vorrichtung nach einem der Ansprüche 1 - 8, wobei der radiale Vorsprung (50) zur Selbstexpandierung ausgestaltet ist.

12. Vorrichtung nach Anspruch 11, wobei der radiale Vorsprung (50) eine Spirale (52) umfasst, die mit einem Abdeckmaterial (54) abgedeckt ist.

13. Vorrichtung nach einem der Ansprüche 1 - 8, wobei der Rahmen (24) Stege umfasst, die an Übergängen (72) miteinander verbunden sind, um Zellen zu definieren, und wobei in einer Nähe der Übergänge mindestens einige der Stege so gestaltet sind, dass sie gewellte und/oder Zick-Zack-Bereiche (70) definieren.

14. Vorrichtung nach Anspruch 13, wobei, indem die Stege so gestaltet sind, dass sie gewellte und/oder Zick-Zack-Bereiche (70) definieren, die wirksame Steglänge der Stege in der Nähe der Übergänge verlängert ist, um in der Nähe der Übergänge auftretende Beanspruchung über eine größere wirksame Länge zu verteilen, als wenn die Stege nicht so gestaltet wären, dass sie die gewellten und/oder Zick-Zack-Bereiche definieren.

15. Vorrichtung nach einem der Ansprüche 1 - 8, wobei der Rahmen (24) Stege umfasst, die an Übergängen (72) miteinander verbunden sind, um Zellen zu definieren, und wobei mindestens einige der Stege so angeordnet sind, dass sie Sätze von zwei oder mehr parallelen Stegen (74) zwischen Übergängen definieren, wobei jeder der Stege in dem Satz eine Breite von weniger als 0,4 mm hat.

## Revendications

1. Appareil pour traiter un sujet ayant une valve mitrale malade, l'appareil comprenant :
un élément d'ancrage (20) conçu pour être implanté dans l'atrium gauche du sujet de sorte qu'aucune partie de l'élément d'ancrage (20) ne s'étende à travers la valve mitrale du sujet, l'élément d'ancrage (20) comprenant :
un anneau (40) ;
un cadre (24) s'étendant vers le haut depuis l'anneau (40), le cadre (24) étant conçu pour ancrer l'élément d'ancrage (20) à l'intérieur de l'atrium gauche, avant que la croissance tissulaire sur l'élément d'ancrage (20) ne se produise, par le déploiement du cadre (24) contre au moins l'un parmi des parois de l'atrium gauche et le plafond de l'atrium gauche ;
une saillie radiale (50) conçue pour s'étendre radialement vers l'extérieur à partir du cadre (24), pour être placée à proximité de l'anneau mitral natif du sujet, et pour produire une croissance tissulaire sur la saillie radiale (50) à partir des parois de l'atrium gauche au moins à proximité de l'anneau mitral natif du sujet ; et
un matériau de liaison (56) disposé entre la saillie radiale (50) et l'anneau (40), le matériau de liaison (56) formant un joint d'étanchéité entre la saillie radiale (50) et l'anneau (40) ; et
un appareil de valve mitrale prothétique conçu pour :
à la suite de la survenue de la croissance à partir des parois de l'atrium gauche sur la saillie radiale (50), être placé au moins partiellement à l'intérieur de l'élément d'ancrage (20), et
s'ancrer à l'élément d'ancrage (20), au moins partiellement en se déployant radialement contre l'anneau (40) .

2. Appareil selon la revendication 1, la saillie radiale (50) étant conçue pour s'étendre radialement vers l'extérieur à partir du cadre (24) à une hauteur de niveau avec l'anneau (40).

3. Appareil selon la revendication 1, la saillie radiale (50) étant conçue pour s'étendre radialement vers l'extérieur à partir du cadre (24) à une hauteur comprise entre 1 mm et 20 mm au-dessus de l'anneau (40).

4. Appareil selon la revendication 1, la saillie radiale (50) étant conçue pour épouser la forme des parois de l'atrium gauche à proximité de l'anneau mitral, de sorte que la saillie radiale (50) remplisse tout espace entre l'extérieur du cadre (24) et les parois de l'atrium gauche à proximité de l'anneau mitral.

5. Appareil selon la revendication 1, une taille de l'anneau (40) étant inférieure à une taille de l'anneau mitral natif du sujet.

6. Appareil selon la revendication 1, la saillie radiale (50) étant conçue pour produire une croissance tissulaire sur la saillie radiale (50) des parois de l'atrium gauche au moins à proximité de l'anneau mitral natif du sujet au cadre (24).

7. Appareil selon la revendication 1, la saillie radiale (50) comprenant une ailette, ou
la saillie radiale (50) comprenant une saillie radiale (50) torique, ou
la saillie radiale (50) ayant une forme elliptique.

8. Appareil selon la revendication 1, la saillie radiale (50) étant conçue pour s'étendre radialement vers l'extérieur à partir du cadre (24) autour de plus de 70 pour cent d'une circonférence du cadre (24).

9. Appareil selon l'une quelconque des revendications 1 à 8, l'élément d'ancrage (20) comprenant en outre une jupe qui recouvre une partie du cadre (24) s'étendant depuis l'anneau (40) jusqu'à une hauteur d'au moins 5 mm depuis l'anneau (40) sur le cadre (24), et le matériau de liaison (56) comprenant une partie de la jupe.

10. Appareil selon la revendication 9, la saillie radiale (50) étant conçue pour produire une croissance tissulaire sur la saillie radiale (50) des parois de l'atrium gauche au moins à proximité de l'anneau mitral natif du sujet à la jupe.

11. Appareil selon l'une quelconque des revendications 1 à 8, la saillie radiale (50) étant conçue pour s'autodéployer.

12. Appareil selon la revendication 11, la saillie radiale (50) comprenant une spirale (52) qui est recouverte d'un matériau de recouvrement (54).

13. Appareil selon l'une quelconque des revendications 1 à 8, le cadre (24) comprenant des entretoises qui sont reliées les unes aux autres au niveau de jonctions (72), de sorte à définir des cellules, et à proximité des jonctions au moins certaines des entretoises étant façonnées pour définir des régions (70) en ondulation et/ou en zigzag.

14. Appareil selon la revendication 13, en étant façonnées pour définir des régions (70) en ondulation et/ou en zigzag, la longueur d'entretoise efficace des entretoises étant allongée à proximité des jonctions, de sorte à répartir une contrainte qui se produit à proximité des jonctions sur une longueur efficace plus grande que si les entretoises n'étaient pas façonnées pour définir les régions en ondulation et/ou en zigzag.

15. Appareil selon l'une quelconque des revendications 1 à 8, le cadre (24) comprenant des entretoises qui sont reliées les unes aux autres au niveau de jonctions (72), de sorte à définir des cellules, et au moins certaines des entretoises étant disposées pour définir des ensembles de deux entretoises parallèles (74) ou plus entre des jonctions, chacune des entretoises dans l'ensemble ayant une largeur inférieure à 0,4 mm.
